# EUROPEAN PATENT SPECIFICATION

(11) **EP 0 527 779 B1**
(45) Date of publication and mention of the grant of the patent: **04.03.1998**
(21) Application number: 91907930.1
(22) Date of filing: 12.04.1991
(51) Int. Cl.: A47K 7/02, A61F 13/36, A61M 35/00, B32B 31/12

(54) **Applicator/wipe pad, array and manufacturing method of such applicator/wipe pads**
Auftrags-/Wischgerät sowie Anordnung und Herstellverfahren solcher Wischgeräte
Tampon d'application et d'essuyage, arrangement et procédé de fabrication de tels tampons

(30) Priority: 13.04.1990 US 508967
(43) Date of publication of application: 24.02.1993
(73) Proprietor: M.J. WOODS, INC., Grand Rapids, MI 49506 (US)
(72) Inventor: WOODS, Marilyn, S., Escondido, CA 92026 (US); WOODS, James, M., Escondido, CA 92026 (US)
(74) Representative: Gold, Tibor Z.
(86) International application number: US9102525
(87) International publication number: WO9115983

(56) References cited:
- GB-A- Q4 609
- GB-A- Q6 160
- US-A- 2 616 428
- US-A- 2 841 811
- US-A- 2 927 335
- US-A- 2 961 677
- US-A- 2 964 772
- US-A- 3 131 410
- US-A- 3 142 855
- US-A- 3 221 359
- US-A- 3 666 611
- US-A- 3 737 939
- US-A- 3 784 998
- US-A- 4 014 616
- US-A- 4 121 386
- US-A- 4 342 613
- US-A- 4 893 956
- US-A- 4 925 453
- US-A- 4 935 087

## Description

The present invention relates to a disposable multilayer laminated pad and a method of making the laminated pad. In particular, the present invention relates to an improved hand held laminated pad suitable as a wipe and/or applicator. The laminated pad has a non-woven base pad, an impervious barrier shield that protects the user from contact with fluids and solids on the base pad and a handle.

Many items are widely used as wipes and applicators for wiping or applying substances from or to surfaces such as those on a human, e.g., skin, finger nails, toe nails, or in a human, e.g., internal organs and bones during an operation. Thus, these items are widely used in both medical and non-medical fields. Small cotton or rayon balls, pads or gauzes are perhaps the most widely used items for these purposes on the market today. Small sponges are also widely used items.

In the medical field, these items are used for cleaning the skin and other surfaces, such as the surfaces of internal organs, by the application of a disinfectant or solvent and/or the wiping away of blood and other fluids, including other body fluids, and other materials. The cotton balls, sponges and gauze pads are grasped between the fingers and applied to the area of concern to wipe away or apply fluids or other materials. One problem with this prior art approach is that the fingers frequently become contaminated with the fluid or other materials that can act as a conduit to transfer the contamination to other people or areas. This is especially a problem in view of the fluid borne diseases such as AIDS and hepatitis.

In the cosmetics and personal care fields, these items are widely used to apply and to remove makeup and to apply other personal care products such as lotions, creams and nail polish remover. Unfortunately, the item transfers the makeup or personal care product to the user's fingers which is often undesirable. For example, when a nail polish remover, which is usually acetone based, is being used it can be transferred to the fingers of the hand holding the item. The nail polish remover can harm the fingers and remove the nail polish from the user's finger nails on the hand holding the item even if removal from these nails was not desired. Also, the nail polish remover can undesirably remove nail polish from a finger nail adjacent to the finger from which the nail polish is being removed. Also, the transfer can result in a waste of the makeup or personal care product.

These items are often amorphous in that they have no defined shape and therefore no defined edges. Thus, these items are not ideally suited to apply or wipe materials to or from surfaces that have an arcuate edge, e.g., finger nails, and from surfaces that have straight edge, e.g., the edge formed between the nose and cheek. Other items only have arcuate or straight edges and are not very effective when surfaces having a different shape are encountered.

One example of the prior art approach is disclosed in US-A-4 053 242. This patent discloses a combination of an applicator and a dispensing package wherein the applicator described as a generally T-shaped disposable product applicator, with a pad portion of the applicator being capable of being folded upon itself which in folded condition lies flat and has a substantially uniform thickness so that it can be stacked in a dispensing container. When removed from the container, the pad portion of each applicator will automatically unfold and assume a product applying configuration. When the applicator pad portion is in its folded condition, the product application surface of the applicator is folded upon itself, and the product which is on or impregnated in the applying surface of the applicator will be protected from contamination.

Another example of applicators is illustrated in US-A-3,737,939. This patent discloses a pad, one of which is a sponge, the other of which is made of multiple sheets, both disclosed as having a generally hexagonal configuration, with a pair of coplanarly disposed applicator support sheets, both of which are slit, with the upper sheet being foldable to serve as a handle, and the second sheet to act as a shield sheet.

The problem to be solved by the invention is to propose an improved laminated pad suitable for use as an applicator or wipe that overcomes at least some of the aforementioned shortcomings.

The invention solves this problem by the features given in claim 1, 5 or 6. Advantageous further developments are described in the dependent claims.

The present invention is directed to a multilayer laminated pad suitable for use as an applicator or wipe. The laminated pad has a base pad, a pliable, impervious shield and an adjustable handle. The base pad has a substantially planar attachment surface and is non-woven, soft, pliable and reversibly absorbent. The impervious shield has a top face and an opposed bottom face, the bottom face being affixed to the attachment surface. The shield is coextensive with the base pad. The adjustable handle is affixed to the top face of the impervious shield.

A preferred laminated pad has edges of the base pad with each edge having a face that is preferably substantially perpendicular to the attachment surface. At least one of these edges is arcuate and at least one of these edges is straight. When the laminated pad is utilized to apply or wipe a material to or from a surface, such as a human body part, the edge of the laminated pad utilized can be selected to have a shape similar to that of the surface. Therefore, the laminated pad can be utilized to more accurately apply or remove a material from the surface without applying or removing material from an adjacent surface.

The material of the base pad is capable of absorbing material such as fluids and releasing the absorbed material under pressure. The requisite pressure to release absorbed material can be generated by forcing the base pad against the surface.

The impervious shield inhibits fluid and/or solid material on or in the base pad from migrating through the impervious shield to the fingers of the user. Thus, contacting of the material with the fingers is avoided. The shield preferably does not extend beyond the edges of the base pad unless additional shielding is desired. The shield also protects the user from fluid and/or solid material from another person.

The adjustable handle permits rotation of the base pad and impervious shield relative to the handle to permit the base pad to conform to the surface while permitting the user to maintain a comfortable grip on the handle.

The laminated pad is pliable and flexible which enables the laminated pad to conform to irregular surfaces and thus utilize the entire applying/wiping surface at the same time. The laminated pad can contain material to be applied to the surface. The base pad can be impregnated with a medicant, cosmetic, solvent or the like during the manufacturing of the laminated pad.

The present invention is also directed to a method of manufacturing the multilayer laminated pad that includes the steps of: providing a sheet-form base pad having a top attachment surface; providing a sheet-form, impervious shield having a bottom face and a top face; applying adhesive to at least one of the attachment surface or the bottom face; providing a handle-producing sheet having a bottom surface; selectably applying an adhesive to at least one of the top face or the bottom surface to cause partial adherence of the shield and handle-producing sheet upon combining the shield and handle-producing sheet; juxtaposing the top attachment surface and the bottom face to combine the base pad and the shield; and juxtaposing the top face and the bottom surface to combine the shield and the handle-producing sheet. The method can include the further step of cutting the combined base pad, shield and handle-producing sheet in the shape of the laminated pad and so that the handle-producing sheet has an adhered segment and a free segment. After the cutting step the free segment of the handle-producing sheet is available to be rotated and utilized as a handle.

The invention will now be further described with reference to the drawings in which
FIG. 1 is a perspective view of an embodiment of the laminated pad;
FIG. 2 is a front elevation view of the laminated pad of FIG. 1;
FIG. 3 is a schematic illustration of a method of manufacture of the laminated pad of FIG. 1;
FIG. 4 is a side elevation view of an array of laminated pads;
FIG. 5 is a perspective view of a preferred embodiment of the laminated pad;
FIG. 6 is a front elevational view of the laminated pad of FIG. 5 taken along line 6-6;
FIG. 7 is a schematic illustration of a preferred method of manufacturing the laminated pad of FIG. 5;
FIG. 8 is a side view of a coater for coating adhesive onto the shield;
FIG. 9 is a side view of a coater for selectively coating adhesive onto the handle-producing sheet;
FIG. 10 is an exploded elevational view of the three sheets used to produce the laminated pad taken along line 10-10 of FIG. 7;
FIG. 11 is a top elevational view of an alternative embodiment of the laminated pad;
FIG. 12 is a side elevational view of the laminated pad of Fig. 11 taken along line 12-12 of FIG. 11; and
FIG. 13 is a top elevational view of an array of laminated pads.

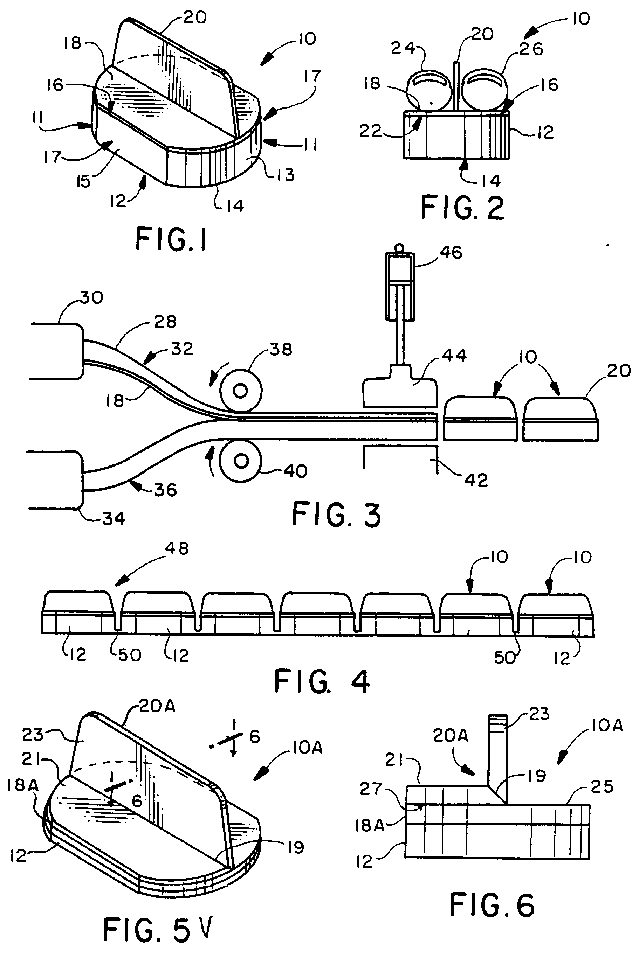

Referring to the drawings, particularly to FIGS. 1 and 2, there is illustrated an embodiment of the laminated pad 10 of the present invention. The laminated pad 10 has a base pad member or portion 12 having a lower or applying/wiping surface 14 and an upper or top, generally parallel and planar attachment surface 16. The base pad 12 is an absorbent pad for absorbing a liquid or holding a material (such as solid powder), either for cleanup or for application of the liquid or material to a surface or the like.

The base pad 12 has at least one arcuate edge 11 having a face 13 that is substantially perpendicular to the attachment surface 16. The base pad 12 also has at least one straight edge 17 having a face 19 that is substantially perpendicular to the attachment surface 16. Most preferably, the base pad 12 has two opposed arcuate edges 11 and two opposed straight edges 17.

The base pad 12 is attached to a bottom face 22 of a fluid and solid impervious shield or barrier 18 and a handle or grasping tab 20, with the shield or barrier 18 having attached thereto the upstanding handle 20.

As shown in FIGS. 5 and 6, the preferred laminated pad 10A has an impervious shield 18A that is coextensive with and affixed to the base pad 12. The handle 20A has a bottom surface 27 which is partially adhered to a top face 25 of the shield 18A to produce an adhered segment 21 and a free segment 23. Free segment 23 is capable of movement along a fold or score 19 to permit rotation of the handle 20A relative to the shield 18A and base pad 12.

The shield 18A is preferably pliable and impervious to the fluids and solids to which the base pad 12 is exposed.

The laminated pad is designed such that it may be conveniently made in various sizes, with one size as illustrated for ease of use in place of the typical cotton balls and the like. To this end, the applicator can be made of a size such that the handle extends upward a distance on the order of about 12 mm (one-half inch) to about 25 mm (one inch) to extend slightly above the typical or average size human fingers 24 and 26, as illustrated in FIG. 2. The laminated pad can have a width of about twice the height of the tab or handle, i.e. about 25 to about 50 mm (about one to about two inches) for a preferred size. The laminated pad can have a length of slightly greater than its width.

This size of the laminated pad and handle provide an easy and convenient size, with a convenient tray of grasping and manipulating the laminated pad between a pair of adjacent fingers, as illustrated. It can also be grasped between the thumb and forefinger or other finger of the hand. The shield or barrier panel protects the hand or fingers against contamination by a liquid or other material contained, absorbed, or contacted by the base pad. The present construction also lends itself to easy and economical construction for the production of simple and inexpensive laminated pads.

Referring to FIG. 3, there is illustrated a schematic diagram of a typical production process and method of making the laminated pads 10 of the present invention. A first extruder 30 extrudes a T-bar form of a member 32, which has an inverted T cross section and can be formed of a thin flexible plastic material, e.g., about 0.02 to 0.03 millimeters (mm). An adjacent and parallel extruder or dispenser 34 provides a continuous elongated member 36 of a suitable base pad material. The members 32 and 36 are fed through a pair of opposed rollers 38 and 40 that are rotated in the directions indicated by their respective arrows. The rollers 38 and 40 bring the two members 32 and 36 together at the mating surfaces and bond them into a combined, unitary structure. This unitary structure proceeds forward and passes over a back up mandrel or the like 42, above which is disposed a reciprocating cutter 44 powered by a suitable hydraulic or air cylinder 46 for cutting the combined structure into the laminated pads 10. The roller 38 biases a central leg 28 of the T-bar 32 downward in a planar position parallel to one side of the shield 18, and the cutter 44 simultaneously cuts the members 32 and 36. As the cutter 44 is removed, the handle 20 can pop or be rotated up. The laminated pads 10 may be totally separated, as illustrated in FIG. 3, into separate and distinct laminated pads 10 or may be formed as an array 48 of detachable laminated pads 10 as shown in FIG. 4. The mandrel 42 and cutter 44 are selected to achieve the desired results.

Referring to FIG. 4, a plurality of the laminated pads are formed in a manner similar to that shown in FIG. 3. However, base pads 12 remain connected together by a thin connector strip 50, such that the nest or array 48 of the laminated pads 10 may be rolled onto a roller or the like and dispensed by tearing off individual laminated pads. In an embodiment that is not illustrated, the array 48 can have a number of rows and columns of laminated pads 10. Alternatively, a number of arrays 48 can be stacked and boxed in a nest-like fashion. A laminated pad is selected for use and the connector strip 50 is torn, separating the selected laminated pad from the adjacent laminated pad(s).

In an alternative embodiment that is not illustrated, the connector strip 50 is formed from the shield or handle.

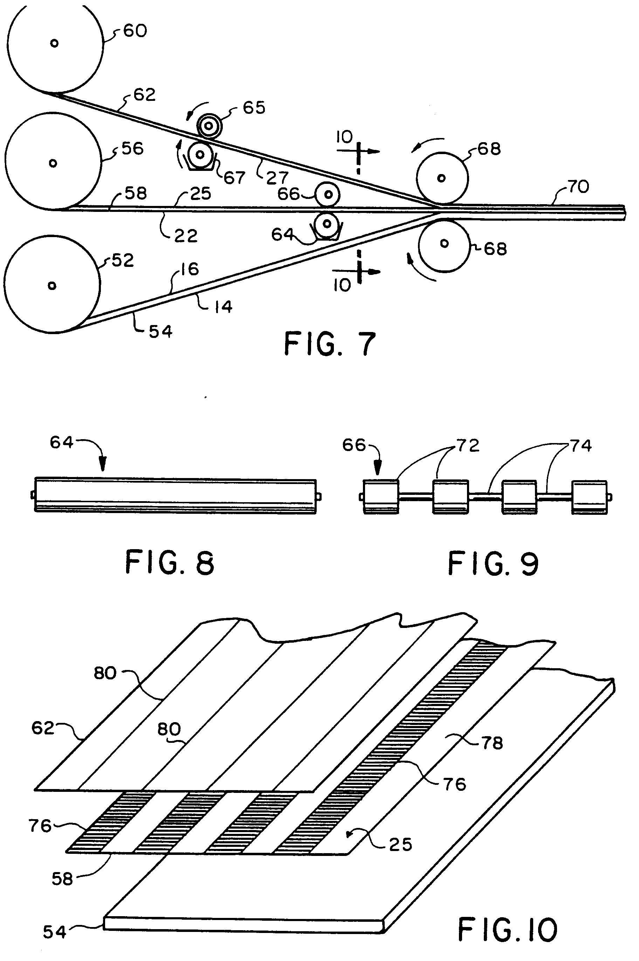

FIG. 7 illustrates a preferred method of producing the laminated pad of the present invention wherein the laminated pad has three layers. A roll 52 provides sheet-formed base pad material 54, a roll 56 provides sheet-form impervious shield material 58 and a roll 60 provides sheet-form handle-producing material 62. A conventional adhesive is coated onto the bottom face 22 of the impervious shield material 58 by a full surface adhesive coater 64. Alternatively, the adhesive can be coated onto the attachment surface 16 of the base pad material. This alternative embodiment is not illustrated. A conventional adhesive is selectively coated onto the top face 25 of the impervious shield material 58 in strips by a partial, adhesive strip-forming coater 66. Alternatively, the adhesive can be selectively coated onto the bottom surface 27 of the handle-producing material 62. This alternative embodiment is not illustrated. In further alternative embodiments, also not illustrated, adhesive is coated onto at least one of the attachment surface 16 and the bottom face 22 and adhesive is selectively coated to at least one of the top face 25 and the bottom surface 27. The materials 54, 58 and 60 are then forced between two opposed rollers 68 whose directions of rotation are indicated by their respective arrows. The rollers 68 provide sufficient compressive force to adhere the materials 54, 58 and 62 to each other and produce a laminated sheet 70. The laminated sheet can then be fed into a mandrel 42 and cutter 44 assembly as shown in FIG. 3 to produce laminated pads. As discussed above, the mandrel and cutter assembly can be selected to produce an array of laminated pads.

Optionally, the handle-producing material 60 can be scored either prior to or after passing between the rollers 68. Scoring can be achieved utilizing a scoring roller 65 and a compression roller 67.

FIG. 8 illustrates the full surface adhesive coater 64 as a roller that provides adhesive to the entire bottom face or attachment surface.

FIG. 9 illustrates the partial, adhesive strip-forming coater 66 that selectively coats strips of adhesive to the top face or bottom surface. The strip-forming coater 66 has adhesive coating sections 72 with non-adhesive coating sections 74 therebetween.

FIG. 10 illustrates the top face 25 of the impervious shield material 58 having adhesive coated sections 76 and uncoated sections 78. The handle-producing material 62 has score lines 80 which are positioned so that when the laminated sheet is cut the handle can be more readily rotated into the desired position.

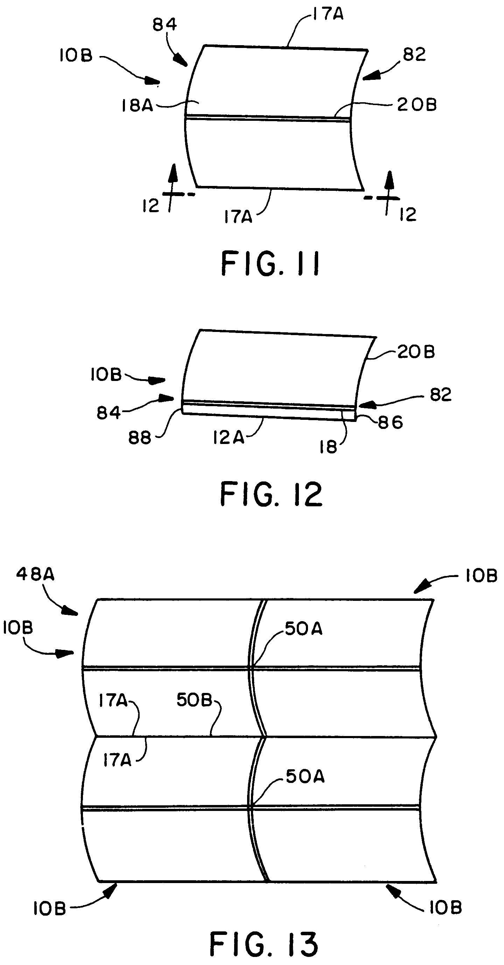

FIGS. 11 and 12 illustrate an alternative embodiment of the laminated pad 10B. The laminated pad 10B has a base pad 12A and a concave arcuate edge 82 and a convex arcuate edge 84. The base pad 12A also has a face 86 of the concave arcuate edge 82 and a face 88 of the convex arcuate edge 84. The concave arcuate edge 82 and face 86 can be utilized on surfaces that have a convex end (not shown). The convex arcuate edge 84 and face 88 can be used on a surface having a concave end (not shown). The laminated pad 10B also has straight edges 17A that are utilized as described above.

FIG. 13 illustrates an array 48A of four laminated pads 10B. It being understood that the number of laminated pads in the array 48A is not limited to four and the array 48A can be symmetrical or unsymmetrical. Connector strips 50A and 50B extend between adjacent laminated pads 10B to maintain the array 48A. Connector strip 50B can be a separable line of demarcation between the straight edges 17A of the laminated pads 10B. The connector strips 50A and 50B permit individual laminated pads 10B to be detached from the array 48A.

The base pad can be made of any number of foam compositions, a woven or non-woven fiber or fabric material, gauze, sponge, and the like. Preferably, the base pad is made of a non-woven material that is soft, pliable and reversibly absorbent. More preferably, the base pad is made of a material that is substantially lintless. The preferred material for the base pad is capable of holding a fluid or solid material and subsequently releasing the fluid or solid material by the application of pressure. The base pad is most preferably made of a conventional entangled cotton which is typically processed with water to cause entanglement of the cotton fibers. A commercially available entangled cotton is Webril from International Paper Co., Veratec Division, Walpole, MA.

The handle and shield can be made of the same material that can be any suitable impervious, non-reactant material, such as a treated paper that is impervious or a suitable thin plastic film material. The material is flexible and has structural integrity, such that it can be easily handled and support its structure and the base pad.

The shield preferably has a thickness on the order of about 0,02 to 0,03 mm.

Preferably, the material that the shield and handle are made of is non-reactive with materials to be applied or removed or to be impregnated into the base pad. The laminated pad can also preferably be made with biodegradable materials.

Suitable materials for the impervious shield include paper impregnated or coated with a latex or resin to render the paper impervious, sheet-form plastic, e.g., polyethylene and polypropylene, and the like. The handle can be made of the same material as the shield provided that the material has enough strength to enable the user to properly grip the handle and apply the desired force to the laminated pad.

Suitable bonding between the shield or barrier and base pad can be achieved with a non-reactive bonding. This may be accomplished by mechanical bonding between the materials, such as electromagnetic, and other forms or adhesives may be utilized.

The adhesive utilized to produce the laminated sheet that is cut into laminated pads can be a conventional adhesive that provides sufficient bonding of the various layers of material and which is substantially non-reactive with, and does not degrade upon exposure to, the materials to which the laminated pad is exposed.

The laminated pad can be used for the application of various materials, such as liquids, pastes, powders and the like to various surfaces, and/or may be utilized for the removal of such materials. The laminated pad can be impregnated with a material to be applied. The laminated pad has many uses, including nail polish removal by the application of acetone and other solvents and the like. It may also be utilized for application and/or removal of facial and other skin cleansers, moisturizers, makeup, etc. It can also be used in the medical field, either for the application of various medications or the like, or for cleansing and the like. The pad can be sterilized for medical use.

## Claims

1. An applicator/wipe pad (10) for applying a liquid or solid substance and for removing a liquid or solid substance by wiping, of the type including an absorbent pad (12), an impervious shield or barrier (18) and a flexible handle (20), the absorbent pad (12) being reversibly absorbent, and having an applying/wiping surface (14), an attachment surface (16) opposite the applying/wiping surface (14), and edges (11, 17) defining an outer periphery;
the impervious shield or barrier (18) having a lower surface (22) affixed to the attachment surface (16) of the absorbent pad (12), an upper surface (25), and edges defining an outer periphery, the outer periphery of the impervious shield or barrier (18) being coextensive with the outer periphery of the absorbent pad (12); the handle (20) and the upper surface (25) being joined together by means of an adhesive; characterized by:
the handle (20) being L-shaped, and having one adhered segment (21) affixed to the upper surface (25) of the impervious shield or barrier (18), and one free segment (23) pivotally connected to the adhered segment (21) along a single straight fold line (19) such that the free segment (23) can be rotated relative to the impervious shield or barrier (18) from a position adjacent to the impervious shield or barrier, the adhered segment (21) and the free segment (23) having edges defining an outer periphery which is coextensive with the outer peripheries of the absorbent pad (12) and of the impervious shield or barrier (18).

2. An applicator/wipe pad (10B) according to claim 1 having four edges, two edges being arcuate, for example one concave arcuate edge (82) and one convex arcuate edge (84), and two edges (17A) being straight.

3. An applicator/wipe pad (10, 10A, 10B) according to claims 1 or 2, wherein the absorbent pad (12) for example is made of woven or non-woven fiber, or fabric material, or gauze, or sponge.

4. An applicator/wipe pad (10, 10A, 10B) according to claims 1, 2 or 3, wherein the impervious shield or barrier (18) comprises a thin plastic film.

5. An array (48) of applicator/wipe pads (10) according to the pad of anyone of the foregoing claims, comprising:
at least first and second applicator/wipe pads (10) each comprising:
a reversibly absorbent pad (12) having an applying/wiping surface (14), an attachment surface (16) opposite the applying/wiping surface (14), and edges (11, 17) defining on outer periphery,
an impervious shield or barrier (18) having a lower surface (22) affixed to the attachment surface (16) of the absorbent pad (12), an upper surface (25), and edges defining an outer periphery, the outer periphery of the impervious shield or barrier (18) being coextensive with the outer periphery of the absorbent pad (12),
an L-shaped flexible handle (20) having an adhered segment (21) affixed to the upper surface (25) of the impervious shield or barrier (18), and a free segment (23) pivotally connected to the adhered segment (21) along a single straight fold line (19) such that the free segment (23) can be rotated relative to the impervious shield or barrier (18) from a position adjacent to the impervious shield or barrier, the adhered segment (21) and the free segment (23) having edges defining an outer periphery which is coextensive with the outer peripheries of the absorbent pad (12) and of the impervious shield or barrier (18), and
a first edge (17A), the first edged (17A) of the first applicator/wipe pad (10B) being adjacent to the first edge of the second applicator/wipe pad (10B); and
a thin tearable connector strip (50) connecting the first edge (17A) of the second applicator/wipe pad (10B), the connector strip (50) being made of at least one of the absorbent pads (12), the impervious shield or barrier (18) and handle (20) of the first and second applicator/wipe pads (10B).

6. A method of manufacturing a plurality of multilayer pads (10) according to the pad of anyone of claims 1 to 4 and suitable for use as applicators or wipes comprising the steps of:
providing sheet-form base pad material (54) having a top attachment surface (16);
providing sheet-form impervious shield or barrier material (58) having a bottom face (22) and a top face (25);
applying adhesive to at least one of the top attachment surfaces (16) of the base pad material (54) or the bottom face (22) of the impervious shield or barrier material (58);
providing sheet-form handle-producing material (62) having a bottom surface (27);
selectively applying adhesive, for example in strips (76), to at least one of the top face (25) of the impervious shield or barrier material (58) or the bottom surface (27) of the handle-producing material (62) to cause adherence of the impervious shield or barrier material (58) and a segment of the handle-producing material (62) to each other where adhesive is applied upon combining of the impervious shield or barrier material (58) and the handle-producing material (62);
juxtaposing the top attachment surface (16) and the bottom face (22) to combine the base pad material (54) and the impervious shield or barrier material (58), and juxtaposing the top face (25) and the bottom surface (27) to combine the impervious shield or barrier material (58) and the said handle-producing material (62) by adhering the impervious shield or barrier material (58) and the handle-producing material (62) to each other where adhesive is applied, for example in strips (76), to produce a laminated sheet (70); and
cutting the laminated sheet (70) to produce individual multilayer pads (10), the cutting being such that each multilayer pad (10) so produced has a handle (20) with an adhered segment (21) affixed to the upper surface (25) of the impervious shield or barrier (18) where adhesive was applied, for example in strips (76), to the top face (25) of the impervious shield or barrier material (58) or the bottom surface (27) of the handle-producing material (62), and a free segment (23) pivotally connected to the adhered segment (21) along a single straight fold line (19).

7. The method of claim 6, which comprises scoring the handle-producing material (62) between the adhered segment (21) and the free segment (23) along the fold line (19).

8. The method of claim 6, which comprises employing an adhesive strip-forming coater (66) having a plurality of adhesive coating sections (72) with non-adhesive coating sections (74) between the adhesive coating sections (72) for selectively applying adhesive in strips (76) to at least one of the top face (25) of the impervious shield or barrier material (58) or the bottom surface (27) of the handle-producing material (62).

## Patentansprüche

1. Applizier-/Wischkissen (10) zum Auftragen einer Flüssigkeit oder einer Festsubstanz und zum Beseitigen einer Flüssigkeit oder einer Festsubstanz durch Abwischen, des Typs, der ein Absorbierkissen (12), eine undurchlässige Abschirmung oder Barriere (18) und einen flexiblen Griff (20) aufweist, wobei das Absorbierkissen (12) reversibel absorbierend ist und eine Applizier-/Wischfläche (14), eine der Applizier-/Wischfläche (14) entgegengesetzte Befestigungsfläche (16) und einen äußeren Umfang definierende Ränder (11, 17) aufweist,
wobei die undurchlässige Abschirmung oder Barriere (18) eine untere Fläche (22), welche an der Befestigungsfläche (16) des Absorbierkissens (12) befestigt ist, eine obere Fläche (25) und einen Außenumfang definierende Ränder aufweist, wobei sich der Außenumfang der undurchlässigen Abschirmung oder Barriere (18) mit dem Außenumfang des Absorbierkissens (12) deckt, wobei der Griff (20) und die obere Fläche (25) mittels eines Klebemittels miteinander verbunden sind, dadurch gekennzeichnet, daß
der Griff (20) L-förmig ist und einen Haftabschnitt (21), welcher an der oberen Fläche (25) der undurchlässigen Abschirmung oder Barriere (18) befestigt ist, und einen Freiabschnitt (23) aufweist, welcher entlang einer einzelnen geraden Faltlinie (19) derart schwenkbar mit dem Haftabschnitt (21) verbunden ist, daß der Freiabschnitt (23) relativ zu der undurchlässigen Abschirmung oder Barriere (18) von einer zu der undurchlässigen Abschirmung oder Barriere benachbarten Position aus drehbar ist, wobei der Haftabschnitt (21) und der Freiabschnitt (23) Ränder aufweisen, welche einen Außenumfang definieren, der sich mit den Außenumfängen des Absorbierkissens (12) und der undurchlässigen Abschirmung oder Barriere (18) deckt.

2. Applizier-/Wischkissen (10B) nach Anspruch 1, mit vier Rändern, von denen zwei Ränder bogenförmig, z.B. ein konkav gekrümmter Rand (82) und ein konvex gekrümmter Rand (84), sind und von denen zwei Ränder (17A) gerade sind.

3. Applizier-/Wischkissen (10, 10A, 10B) nach Anspruch 1 oder 2, wobei das Absorbierkissen (12) z.B. aus gewebtem oder nicht gewebtem Fasermaterial oder Gewebematerial oder Gaze oder Schwamm ist.

4. Applizier-/Wischkissen (10, 10A, 10B) nach einem der Ansprüche 1, 2 oder 3, wobei die undurchlässige Abschirmung oder Barriere (18) einen dünnen Kunststoffilm aufweist.

5. Reihe (48) von Applizier-/Wischkissen (10) gemäß dem Kissen nach einem der vorhergehenden Ansprüche, mit
wenigstens einem ersten und einem zweiten Applizier-/Wischkissen (10), von denen jedes aufweist:
ein reversibel absorbierendes Kissen (12) mit einer Applizier-/Wischfläche (14), einer der Applizier-/Wischfläche (10) entgegengesetzten Befestigungsfläche (16) und einen Außenumfang definierenden Rändern (11, 17),
eine undurchlässige Abschirmung oder Barriere (18) mit einer unteren Fläche (22), welche an der Befestigungsfläche (16) des Absorbierkissens (12) befestigt ist, einer oberen Fläche (25) und einen Außenumfang definierenden Rändern, wobei sich der Außenumfang der undurchlässigen Abschirmung oder Barriere (18) mit dem Außenumfang des Absorbierkissens (12) deckt,
einen L-förmigen, flexiblen Griff (20) mit einem Haftabschnitt (21), welcher an der oberen Fläche (25) der undurchlässigen Abschirmung oder Barriere (18) befestigt ist, und einem Freiabschnitt (23), welcher entlang einer einzelnen geraden Faltlinie (19) derart schwenkbar mit dem Haftabschnitt (21) verbunden ist, daß der Freiabschnitt (23) von einer zu der undurchlässigen Abschirmung oder Barriere benachbarten Position aus relativ zu der undurchlässigen Abschirmung oder Barriere (18) drehbar ist, wobei der Haftabschnitt (21) und der Freiabschnitt (23) Ränder aufweisen, welche einen Außenumfang definieren, der sich mit den Außenumfängen des Absorbierkissens (12) und der undurchlässigen Abschirmung oder Barriere (18) deckt, und
einen ersten Rand (17A), wobei der erste Rand (17A) des ersten Applizier-/Wischkissens (10B) benachbart zu dem ersten Rand des zweiten Applizier-/Wischkissens (10B) angeordnet ist, und
einem dünnen, zerreißbaren Verbindungsstreifen (50), welcher den ersten Rand (17A) des zweiten Applizier-/Wischkissens (10B) anschließt, wobei der Verbindungsstreifen (50) von wenigstens dem Absorbierkissen (12), der undurchlässigen Abschirmung oder Barriere (18) oder dem Griff (20) des ersten und des zweiten Applizier-/Wischkissens (10B) ausgebildet ist.

6. Verfahren zum Herstellen einer Mehrzahl von für die Verwendung als Appliziermittel oder Wischer geeigneten Mehrschichtkissen (10) gemäß dem Kissen nach einem der Ansprüche 1 bis 4, mit den Schritten:
Vorsehen von blattförmigem Basiskissenmaterial (54) mit einer oberen Befestigungsfläche (16),
Vorsehen von blattförmigem, undurchlässigem Abschirmungs- oder Barrierematerial (58) mit einer unteren Fläche (22) und einer oberen Fläche (25),
Auftragen von Klebemittel auf wenigstens die obere Befestigungsfläche (16) des Basiskissenmaterials (54) oder die untere Fläche (22) des undurchlässigen Abschirmungs- oder Barrierematerials (58),
Vorsehen von blattförmigem Griffherstellungsmaterial (62) mit einer unteren Fläche (27),
selektives Auftragen von Klebemittel, beispielsweise in Streifen (76), auf wenigstens die obere Fläche (25) des undurchlässigen Abschirmungs- oder Barrierematerials (58) oder die untere Fläche (27) des Griffherstellungsmaterials (62), um auf das Verbinden des undurchlässigen Abschirmungs- oder Barrierematerials (58) und des Griffherstellungsmaterials (62) eine Haftung des undurchlässigen Abschirmungs- oder Barrierematerials (58) und eines Abschnitts des Griffherstellungsmaterials (62) aneinander zu bewirken, wo Klebemittel aufgebracht ist,
Zusammenbringen der oberen Befestigungsfläche (16) und der unteren Fläche (22) zum Verbinden des Basiskissenmaterials (54) und des undurchlässigen Abschirmungs- oder Barrierematerials (58) und Zusammenbringen der oberen Fläche (25) und der unteren Fläche (27) zum Verbinden des undurchlässigen Abschirmungs- oder Barrierematerials (58) und des Griffherstellungsmaterials (62) durch Aneinanderkleben des undurchlässigen Abschirmungs- oder Barrierematerials (58) und des Griffherstellungsmaterials (62), wo Klebemittel beispielsweise in Streifen (76) aufgetragen ist, um ein laminiertes Blatt (70) herzustellen, und
Abschneiden des laminierten Blatts (70) zum Herstellen individueller Mehrschichtkissen (10), wobei das Abschneiden derart ist, daß jedes derart hergestellte Mehrschichtkissen (10) einen Griff (20) mit einem Haftabschnitt (21), welcher an der oberen Fläche (25) der undurchlässigen Abschirmung oder Barriere (18) befestigt ist, wo Klebemittel beispielsweise in Streifen (76) auf die obere Fläche (25) des undurchlässigen Abschirmungs- oder Barrierematerials (58) oder die untere Fläche (27) des Griffherstellungsmaterials (62) aufgebracht wurde, und einem Freiabschnitt (23) aufweist, welcher entlang einer einzelnen geraden Faltlinie (19) schwenkbar mit dem Haftabschnitt (21) verbunden ist.

7. Verfahren nach Anspruch 6, bei welchem das Griffherstellungsmaterial (62) entlang der Faltlinie (19) zwischen dem Haftabschnitt (21) und dem Freiabschnitt (23) eingekerbt wird.

8. Verfahren nach Anspruch 6, bei welchem ein Klebemittel-Streifenbildungs-Beschichter (66) verwendet wird, welcher eine Mehrzahl von Klebemittel-Beschichtungsabschnitten (72) mit zwischen den Klebemittel-Beschichtungsabschnitten (72) vorgesehenen klebmittelfreien Beschichtungsabschnitten (74) zum selektiven Auftragen von Klebemittel in Streifen (76) auf wenigstens die Oberfläche (25) des undurchlässigen Abschirmungs- oder Barrierematerials (58) oder die untere Fläche (27) des Griffherstellungsmaterials (62) aufweist.

## Revendications

1. Tampon applicateur/d'essuyage (10) destiné à appliquer une substance liquide ou solide et à retirer une substance liquide ou solide par essuyage, du type comprenant un tampon absorbant (12), une protection ou barrière imperméable (18) et une poignée flexible (20), le tampon absorbant (12) étant absorbant de manière réversible et ayant une surface d'application/essuyage (14), une surface de fixation (16) opposée à la surface d'application/essuyage (14) et des bords (11, 17) définissant une périphérie extérieure ;
la protection ou barrière imperméable (18) ayant une surface inférieure (22) fixée à la surface de fixation (16) du tampon absorbant (12), une surface supérieure (25) et des bords définissant une périphérie extérieure, la périphérie extérieure de la protection ou barrière imperméable (18) étant coextensive avec la périphérie extérieure du tampon absorbant (12) ; la poignée (20) et la surface supérieure (25) étant réunies l'une à l'autre au moyen d'un adhésif ; caractérisé en ce que :
la poignée (20) a une forme en L et est pourvue d'un segment collé (21) qui est fixé à la surface supérieure (25) de la protection ou barrière imperméable (18) et d'un segment libre (23) monté pivotant sur le segment collé (21), le long d'une ligne de pliage droite unique (19), de sorte que le segment libre (23) peut être mis en rotation par rapport à la protection ou barrière imperméable (18), depuis une position adjacente à la protection ou barrière imperméable, le segment collé (21) et le segment libre (23) ayant des bords définissant une périphérie extérieure qui est coextensive avec les périphéries extérieures du tampon absorbant (12) et de la protection ou barrière imperméable (18).

2. Tampon applicateur/d'essuyage (10B) selon la revendication 1 qui est pourvu de quatre bords, à savoir deux bords arqués, par exemple un bord arqué concave (82) et un bord arqué convexe (84), et deux bords droits (17A).

3. Tampon applicateur/d'essuyage (10, 10A, 10B) selon la revendication 1 ou 2, dans lequel le tampon absorbant (12) est par exemple constitué de fibres tissées ou non tissées, d'un matériau en tissu, de gaze ou d'éponge.

4. Tampon applicateur/d'essuyage (10, 10A, 10B) selon les revendications 1, 2 ou 3, dans lequel la protection ou barrière imperméable (18) est constituée d'un film plastique mince.

5. Ensemble (48) de tampons applicateurs/d'essuyage (10) conformes au tampon selon l'une quelconque des revendications précédentes, comprenant :
au moins un premier et un second tampons applicateurs/d'essuyage (10) comprenant chacun :
un tampon absorbant de manière réversible (12) pourvu d'une surface d'application/essuyage (14), d'une surface de fixation (16) opposée à la surface d'application/essuyage (14) et de bords (11, 17) définissant une périphérie extérieure,
une protection ou barrière imperméable (18) pourvue d'une surface inférieure (22) fixée à la surface de fixation (16) du tampon absorbant (12), d'une surface supérieure (25) et de bords définissant une périphérie extérieure, la périphérie extérieure de la protection ou barrière imperméable (18) étant coextensive avec la périphérie extérieure du tampon absorbant (12),
une poignée flexible en forme de L (20) pourvue d'un segment collé (21) qui est fixé à la surface supérieure (25) de la protection ou barrière imperméable (18) et d'un segment libre (23) qui est monté pivotant sur le segment collé (21), le long d'une ligne de pliage droite unique (19), de sorte que le segment libre (23) peut être mis en rotation par rapport à la protection ou barrière imperméable (18), depuis une position adjacente à la protection ou barrière imperméable, le segment collé (21) et le segment libre (23) ayant des bords définissant une périphérie extérieure qui est coextensive avec les périphéries extérieures du tampon absorbant (12) et de la protection ou barrière imperméable (18), et
un premier bord (17A), le premier bord (17A) du premier tampon applicateur/d'essuyage (10B) étant adjacent au premier bord du second tampon applicateur/d'essuyage (10B) ; et
une bande de connexion mince déchirable (50) connectant le premier bord (17A) du second tampon applicateur/d'essuyage (10B), la bande de connexion (50) étant constituée au moins des tampons absorbants (12), de la protection ou barrière imperméable (18) ou de la poignée (20) des premier et second tampons applicateurs/d'essuyage (10B).

6. Procédé de fabrication d'une pluralité de tampons multicouches (10) conformes au tampon selon l'une quelconque des revendications 1 à 4, et convenant à une utilisation comme applicateurs ou moyens d'essuyage, comprenant les étapes consistant :
à fournir un matériau de base (54) pour tampon, en forme de feuille, qui est pourvu d'une surface de fixation supérieure (16) ;
à fournir un matériau imperméable (58) pour protection ou barrière, en forme de feuille, qui est pourvu d'une face inférieure (22) et d'une face supérieure (25) ;
à appliquer un adhésif au moins à la surface de fixation supérieure (16) du matériau de base pour tampon (54) ou à la face inférieure (22) du matériau imperméable (58) pour protection ou barrière ;
à fournir un matériau en forme de feuille (62) produisant une poignée, qui est pourvu d'une surface inférieure (27) ;
à appliquer sélectivement un adhésif, par exemple sous forme de bandes (76), au moins à la face supérieure (25) du matériau imperméable (58) pour protection ou barrière ou à la surface inférieure (27) du matériau (62) produisant une poignée, pour faire adhérer le matériau imperméable (58) pour protection ou barrière et un segment du matériau (62) produisant une poignée l'un à l'autre aux endroits où l'adhésif est appliqué, lors de la combinaison du matériau imperméable (58) pour protection ou barrière et du matériau (62) produisant une poignée ;
à juxtaposer la surface de fixation supérieure (16) et la face inférieure (22) pour combiner le matériau de base pour tampon (54) et le matériau imperméable (58) pour protection ou barrière, et à juxtaposer la face supérieure (25) et la surface inférieure (27) pour combiner le matériau imperméable (58) pour protection ou barrière et ledit matériau (62) produisant une poignée, en faisant adhérer le matériau imperméable (58) pour protection ou barrière et le matériau (62) produisant une poignée l'un à l'autre aux endroits où l'adhésif est appliqué, par exemple sous forme de bandes (76), pour produire une feuille stratifiée (70) ; et
à découper la feuille stratifiée (70) pour produire des tampons multicouches individuels (10), la découpe étant telle que chaque tampon multicouches (10) ainsi produit est pourvu d'une poignée (20), un segment collé (21) étant fixé à la surface supérieure (25) de la protection ou barrière imperméable (18) aux endroits où l'adhésif a été appliqué, par exemple sous forme de bandes (76), à la face supérieure (25) du matériau imperméable (58) pour protection ou barrière ou à la surface inférieure (27) du matériau (62) produisant une poignée, et un segment libre (23) étant monté pivotant sur le segment collé (21), le long d'une ligne de pliage droite unique (19).

7. Procédé selon la revendication 6, comprenant le marquage d'un pli dans le matériau (62) produisant une poignée, entre le segment collé (21) et le segment libre (23), le long de la ligne de pliage (19).

8. Procédé selon la revendication 6, comprenant l'utilisation d'un applicateur (66) d'adhésif en forme de bandes, pourvu d'une pluralité de sections d'enduction d'adhésif (72), des sections n'appliquant pas d'adhésif (74) étant prévues entre les sections d'enduction d'adhésif (72) pour appliquer sélectivement un adhésif sous forme de bandes (76) au moins à la face supérieure (25) du matériau imperméable (58) pour protection ou barrière ou à la surface inférieure (27) du matériau (62) produisant une poignée.
